(19) Europäisches Patentamt
European Patent Office
Office européen des brevets

(11) **EP 4 323 040 B1**

(12) **EUROPÄISCHE PATENTSCHRIFT**

(45) Veröffentlichungstag und Bekanntmachung des Hinweises auf die Patenterteilung:
**29.10.2025 Patentblatt 2025/44**

(21) Anmeldenummer: **22719821.5**

(22) Anmeldetag: **29.03.2022**

(51) Internationale Patentklassifikation (IPC):
*A61M 15/00* (2006.01)    *A61M 16/08* (2006.01)
*B05B 1/26* (2006.01)     *B05B 1/28* (2006.01)
*B05B 1/30* (2006.01)     *B05B 7/00* (2006.01)
*B65D 83/30* (2025.01)    *A61M 16/06* (2006.01)

(52) Gemeinsame Patentklassifikation (CPC):
**A61M 15/009; A61M 15/002; A61M 15/0021;
A61M 15/0086; A61M 16/0808; B05B 1/262;
B05B 1/28; B05B 1/3006; B05B 7/0012;
B65D 83/30;** A61M 15/0015; A61M 15/0016;
A61M 16/06; A61M 2205/0211; A61M 2206/14

(86) Internationale Anmeldenummer:
**PCT/EP2022/058292**

(87) Internationale Veröffentlichungsnummer:
**WO 2022/218695 (20.10.2022 Gazette 2022/42)**

(54) **INHALATIONSVORRICHTUNG**

INHALATION DEVICE

DISPOSITIF D'INHALATION

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **15.04.2021 DE 102021203756**

(43) Veröffentlichungstag der Anmeldung:
**21.02.2024 Patentblatt 2024/08**

(73) Patentinhaber: **Werrta GmbH Düsen- und
Zerstäubungstechnik
14089 Berlin (DE)**

(72) Erfinder:
• **RENTSCH, Rüdiger
14089 Berlin (DE)**
• **ETZOLD, Mathias
13581 Berlin (DE)**

(74) Vertreter: **Kehl, Ascherl, Liebhoff & Ettmayr
Patentanwälte Partnerschaft mbB
Emil-Riedel-Straße 18
80538 München (DE)**

(56) Entgegenhaltungen:
WO-A1-2017/118995    WO-A1-2020/165356
WO-A1-2020/260903    DE-U1- 202021 002 521
US-A- 3 069 097      US-A- 4 940 051
US-A- 5 533 498      US-A1- 2011 192 397
US-A1- 2013 306 061  US-B2- 8 333 190

**Beschreibung**

Die vorliegende Erfindung betrifft eine Inhalationsvorrichtung, die einen Inhalationsaktuator aufweist.

Als Inhalationsaktuator wird der Teil einer Inhalationsvorrichtung bezeichnet, den der Anwender der Inhalationsvorrichtung handhabt, um Inhalat zum Inhalieren aus einer Inhalatvorlage zu entnehmen. Handelt es sich bei der Inhalationsvorrichtung um einen Druckgasinhalator, landläufig auch als Inhalationsspray, Inhalierspray oder (aufgrund einer pro Sprühstoß begrenzt ausgegebenen Inhalatmenge) als Dosieraerosol bezeichnet, so wirkt der meist als Einheit von Mundstück und Sprühdosenaufnahme ausgebildete Inhalationsaktuator mit der als Sprühdose ausgebildeten Inhalatvorlage zusammen, indem der Anwender beispielsweise Sprühdose und Inhalationsaktuator gegeneinander drückt.

Ein Nachteil herkömmlicher Druckgasinhalatoren ist die hohe Austragungsgeschwindigkeit des aus dem Inhalat gebildeten Aerosols (Sprays), welches in der Regel aus einer Inhalatdüse durch das Mundstück direkt in Richtung Mund abgegeben wird. Die hohe Austragungsgeschwindigkeit des Sprays (in der Größenordnung von 2 bis 10 m/s) bei zugleich kurzer Austragungszeit (in der Größenordnung von 0,2 s) setzen ein sehr kontrolliertes Verhalten des Anwenders voraus. Die Koordination der manuellen Bedienung des Inhalationsaktuators mit dem Atemzug wird hier zu einem entscheidenden Faktor für die Wirksamkeit des im Inhalat enthaltenen Arzneimittels, der den Erfolg der Therapie entscheiden kann. Denn nur, wenn der kurze Sprühstoß während eines Atemzugs und weder zu früh noch zu spät erfolgt, kann auch ein hinreichender Anteil des ausgetragenen Inhalats in die tieferen Atemwege des Anwenders gelangen.

Um diese Probleme für den Anwender zu entschärfen, werden sogenannte Spacer als Inhalationshilfen eingesetzt. Dabei handelt es sich um eine zwischen Inhalationsaktuator und Anwender anzuordnende Kammer, in der sich ausgetragenes Aerosol sammeln kann, welches der Anwender durch seinen Atemzug aus der Kammer saugt. Die Wirkung der Kammer beruht dabei auf dem Bereitstellen eines relativ großen Luftvolumens in der Kammer, welches zusammen mit der von einer Strahlaufweitung herrührenden Diffusorwirkung für ein Abbremsen des mit dem Sprühstoß ausgestoßenen Aerosols sorgt. Derartige Spacer werden oft als unhandlich aufgrund ihrer Größe und umständlich in der Handhabung empfunden, da sie zunächst auf das Mundstück des Inhalationsaktuators aufgesteckt werden müssen.

Bei einer aus US 3,069,097 bekannten Inhalationsvorrichtung tritt nur das Treibmittel (z.B. auch Luft) aus einer Düse aus, die Wirkstoff-Flüssigkeit wird vom Treibmittel aus einer Kapillare gerissen, und der jeweils mitgeschleppte Tropfen gegen eine Prallkugel geschleudert. Bei eine in US 4,940,051 offenbarten Inhalationsvorrichtung tritt eine Suspension aus; welche einen Wirkstoff in Form von Festkörperpartikeln enthält, wobei die Flüssigkeit beim Austrittaus der Düse verdampft. WO 2020/260903 A1 offenbart eine Aerosolkammer die in den Aktuator einer Inhalationsvorrichtung integriert ist. In der Aerosolkammer ist ein strömungsleitendes Element vorgesehen, das eine Führung des Aerosolstroms weg von der Wand der Aerosolkammer bewirken soll. US 2013/0306061 A1 offenbart einen Prallstrahl, d.h. einen entgegen der Ausströmrichtung der Inhalatdüse gerichteten Luftstrom anstelle eines Prallelements. US 5,533,498 offenbart einen Dosierinhalator mit einer integrierten Deagglomerationskammer. WO 2020/165356 A1 offenbart eine Inhalationsvorrichtung nach dem Oberbegriff vom Anspruch 1. DE 202021002521 U1 offenbart eine Inhalationsvorrichtung nach Anspruch 1, ist aber für den Gegenstand dieses Anspruchs kein Stand der Technik.

Erfindungsgemäß können diese Probleme aus dem Stand der Technik dadurch gelöst werden, dass eine Aerosolkammer in den Inhalationskatuator integriert wird, und die mittlere Tröpfchengeschwindigkeit im aus einer Inhalatdüse als Flüssigkeitsstrahl ausgestoßenen und freiem Zerfall in Tropfen unterliegendem Inhalat durch Verwendung eines Prallelements zur Prallzerstäubung reduziert wird. Die Verwendung des Prallelements gestattet hierbei eine entsprechend kompakte Gestaltung der Aerosolkammer, da in einer derartigen Anordnung kein großes Luftvolumen zum Abbremsen eines Aerosolstroms benötigt wird. Aus der Aerosolkammer kann der Anwender das zerstäubte Inhalat inhalieren. Die geringere Tröpfchengröße durch das Zerprallen kann für eine größere Stabilität des Aerosols in der Aerosolkammer sorgen, d.h. insbesondere weniger Tröpfchenniederschlag an den inneren Wandungen der Aerosolkammer und für eine relativ geringe Neigung der Aerosoltröpfchen, sich miteinander zu größeren Tröpfchen zu vereinigen.

Allgemein stellt die vorliegende Erfindung eine Inhalationsvorrichtung gemäß Anspruch 1 bereit. Vorteilhafte Ausführungsformen können gemäß einem der Ansprüche 2-15 gestaltet sein.

Die Inhalationsvorrichtung weist als Inhalatvorlage, vorzugsweise einen Druckbehälter auf. Die Erfindung ist jedoch vorteilhaft nicht nur umsetzbar, wenn das Inhalat durch den Gasdruck einer Spraydose ausgetragen wird, sondern auch mit einer Handpumpe, Druckerzeugung durch Federkraft oder elektrischer Druckerzeugung.

Der Druckbehälter kann dabei vorteilhafterweise mit einem Dosierventil ausgestattet sein, wie es per se beispielsweise von herkömmlichen Dosiersprays her bekannt ist.

Der Austragsstutzen der Inhalatvorlage kann vorteilhaft in Richtung einer Längsachse des Druckbehälters orientiert sein, und zumindest der überwiegende Teil des Innenvolumens der Aerosolkammer innerhalb einer Projektion des Druckbehälters in Richtung seiner Längsachse angeordnet sein. Ist das Innenvolumen der Aerosolkammer variabel, so gilt das Kriterium dieser Ausführungsform für die maximale Ausdehnung der Aerosolkammer, vorzugsweise für die maximale und die minimale Ausdehnung der Aerosolkammer.

Gemäß einer, insbesondere in Hinblick auf erzielbare Kompaktheit, besonders vorteilhaften Ausführungsform weist die Inhalatvorlage, einen Druckbehälter mit einer Längsachse und einen in Richtung der Längsachse orientierten Austragsstutzen auf, wobei zumindest der überwiegende Teil des Innenvolumens der Aerosolkammer innerhalb einer Projektion des Druckbehälters in Richtung seiner Längsachse angeordnet ist. Ist das Innenvolumen der Aerosolkammer variabel, so gilt dieses Kriterium für die maximale Ausdehnung der Aerosolkammer, vorzugsweise für die maximale und die minimale Ausdehnung der Aerosolkammer.

Gemäß einer vorteilhaften Ausführungsform der erfindungsgemäßen Inhalationsvorrichtung weist der Inhalationsaktuator einen Einsatz auf, der mindestens eine Wandung der Aerosolkammer bildet und das Prallelement trägt. In einer vorteilhaften Weiterbildung dieser Ausführungsform kann der Einsatz die (gesamte) Aerosolkammer ausbilden. Mithilfe eines Einsatzes entsprechend dieser Ausführungsform bzw. ihrer Weiterbildung kann ein herkömmlicher Inhalationsaktuator erfindungsgemäß umgerüstet werden, um eine erfindungsgemäße Inhalationsvorrichtung zu erhalten.

Gemäß einer alternativen vorteilhaften Ausführungsform der erfindungsgemäßen Inhalationsvorrichtung sind die Inhalatzuführung und die Aerosolkammer gemeinsam einstückig ausgeführt, was eine kompakte Bauform und sichere Handhabung begünstigt.

Vorzugsweise sind in einer erfindungsgemäßen Inhalationsvorrichtung die mindestens eine Düsenaustrittsöffnung und der Aerosolaustritt relativ zueinander so angeordnet, dass Inhalat nicht geradlinig vom Düsenaustritt zum Aerosolaustritt strömen kann.

Gemäß vorteilhafter Ausführungsformen der erfindungsgemäßen Inhalationsvorrichtung sind die Hauptaustragsrichtung der mindestens einen Düsenaustrittsöffnung und die Hauptaustragsrichtung des Aerosolaustritts versetzt zueinander angeordnet, und/oder die Hauptaustragsrichtung der mindestens einen Düsenaustrittsöffnung und die Hauptaustragsrichtung des Aerosolaustritts stehen in einem Winkel größer null, vorzugsweise größer 29° und vorzugsweise kleiner 180°, besonders bevorzugt kleiner 151° zueinander. Dabei ist die Hauptaustragsrichtung jeweils als Senkrechte zum Flächenschwerpunkt der jeweils kleinsten durchströmbaren Fläche der Düsenaustrittsöffnung bzw. des Aerosolaustritts definiert.

Vorzugsweise ist in einer erfindungsgemäßen Inhalationsvorrichtung die Luftzufuhr mit einem Luftzufuhrventil, beispielsweise einem sogenannten Flatterventil ausgestattet.

Gemäß einer weiteren vorteilhaften Ausführungsform der erfindungsgemäßen Inhalationsvorrichtung ist der Aerosolaustritt mit einem Aerosolaustrittsventil ausgestattet.

Gemäß einer weiteren vorteilhaften Ausführungsform der erfindungsgemäßen Inhalationsvorrichtung weist der Inhalationsaktuator ein Mundsück auf, wobei der Aerosolaustritt vorzugsweise zwischen Aerosolkammer und Mundstück angeordnet ist. Eine Variante, bei welcher der Inhalationsaktuator einen Einsatz aufweist, der mindestens eine Wandung der Aerosolkammer oder die gesamte Aerosolkammer bildet und das Prallelement trägt, kann vorteilhaft so ausgebildet sein, dass der Einsatz in das Mundstück bzw. durch das Mundstück eingeschoben werden kann.

Unabhängig von der erfindungsgemäßen Ausbildung einer Aerosolkammer mit einem eine lokale Verengung aufweisenden Aerosolaustritt, kann ein Einsatz, der ein Prallelement trägt, vorteilhaft dazu eingesetzt werden, einen Inhalationsaktuator mit einem Prallelement auszustatten, insbesondere auch mit einer Auslegung, wonach aus mindestens einer Düsenaustrittsöffnung austretendes Inhalat vor dem Auftreffen auf das Prallelement in Tröpfchen zerfällt (freier Strahlzerfall in Tropfen, insbesondere auch unabhängig von zusätzlichen Gasströmungen), und nicht erst ein kontinuierlicher Inhalatstrahl am Prallelement zerstäubt, wie später hierin beschrieben.

Gemäß einer weiteren vorteilhaften Ausführungsform der Erfindung ist ein Einsatz in das Mundstück eines Inhalationsaktuators eingeschoben, wobei der Einsatz folgendes aufweist: Zentriermittel zum Zentrieren des Einsatzes im Mundstück, eine quer zur Einschieberichtung angeordnete Wandung, welche in Einschieberichtung vor den Zentriermitteln angeordnet ist, eine in der Wandung vorgesehene Inhalateintrittsöffnung, ein Prallelement, welches entgegen der Einschieberichtung vor der Inhalateintrittsöffnung angeordnet ist, eine quer zur Einschieberichtung neben dem Prallelement angeordnete, in Einschieberichtung frei durchströmbare Fläche, einen in Einschieberichtung vor der frei durchströmbaren Fläche angeordneten Lufteinlass, und einen entgegen der Einschieberichtung vor der frei durchströmbaren Fläche angeordneten Aerosolaustritt. Gemäß einer vorteilhaften Ausführungsform des Einsatzes sind die Zentriermittel integral mit einer durchgehenden oder unterbrochenen Röhre ausgeführt, wobei die Längsrichtung der Röhre der Einschieberichtung entspricht. Entgegen der Einschieberichtung bilden die Wandung und die Röhre somit einen topf-, kammer- oder korbartigen Raum, in dem sich das Prallelement befindet. Vorteilhafterweise kann die Röhre auf ihrer in Längsrichtung der Wandung gegenüberliegenden Seite einen den Aerosolaustritt verengenden Rand aufweisen. Der Lufteinlass kann zumindest teilweise als Unterbrechung in der Röhre ausgebildet und/oder zumindest teilweise in der Wandung ausgeführt sein. Gemäß einer vorteilhaften Ausführungsform des Einsatzes ist die in Einschieberichtung durchströmbare Querschnittsfläche des Aerosolaustritts (Austrittsfläche) kleiner ist als die frei durchströmbare Fläche neben dem Prallelement. Gemäß einer weiteren vorteilhaften Ausführungsform des Einsatzes weist dieser in Einschieberichtung vor der Inhalateintrittsöffnung einen Inhalateintrittsstutzen auf.

Gemäß einer vorteilhaften Weiterbildung der erfindungsgemäßen Inhalationsvorrichtung weist der Inhalationsaktuator eine Auffangvorrichtung zum Auffangen von Inhalat auf, das von dem Prallelement abtropft oder abfließt und/oder sich in der Aerosolkammer niederschlägt. Eine Variante, bei welcher der Inhalationsaktuator einen Einsatz aufweist, der mindestens eine Wandung der Aerosolkammer oder die gesamte Aerosolkammer bildet und das Prallelement trägt, kann vorteilhaft so ausgebildet sein, dass der auch die Auffangvorrichtung oder einen Teil der Auffangvorrichtung bildet.

Vorzugsweise weist in einer erfindungsgemäßen Inhalationsvorrichtung die Auffangvorrichtung ein saugfähiges Materialstück, beispielsweise ein Vlies, Schwämmchen, Zeolith oder dgl. auf.

Gemäß einer vorteilhaften Ausführungsform der erfindungsgemäßen Inhalationsvorrichtung erstreckt sich die maximale Ausdehnung des Inhalationsaktuators parallel zum bestimmungsgemäß in die Aufnahme eingeführten Austragsstutzen.

Gemäß einer, insbesondere in Hinblick auf die erzielbare Kompaktheit, besonders vorteilhaften Ausführungsform der erfindungsgemäßen Inhalationsvorrichtung sind zumindest 10 Prozent, vorzugsweise zumindest 25 Prozent des Innenvolumens der Aerosolkammer bezogen auf eine Richtung, die durch eine gerade Verbindungslinie von der Düsenaustrittsöffnung (bzw. zumindest einer der Düsenaustrittssöffnungen) zum Flächenschwerpunkt der Austrittsfläche des Aerosolaustritts definiert ist, hinter der (entsprechenden) Düsenaustrittsöffnung angeordnet. Ist das Innenvolumen der Aerosolkammer variabel, so gilt das Kriterium dieser Ausführungsform für die maximale Ausdehnung der Aerosolkammer, vorzugsweise für die maximale und die minimale Ausdehnung der Aerosolkammer.

Generell wird im Zusammenhang mit der vorliegenden Erfindung der Ort der kleinsten vom Aerosol durchströmbaren Fläche des Aerosolaustritts als Austrittsfläche betrachtet. Ist dieser Ort nicht eindeutig definiert, beispielsweise wenn der Aerosolaustritt röhrenförmig ausgeführt ist oder keine lokale Verengung aufweist (also beispielsweise die gesamte Aerosolkammer als einseitig offene Röhre ausgeführt ist), so ist unter den kleinsten vom Aerosol durchströmbaren Flächen des Aersosolaustritts die am weitesten anwenderseitig angerordnete die Austrittsfläche. Dem Innenvolumen der Aerosolkammer wird das gesamte vom Aerosol durchströmbare Volumen zwischen Düsenaustrittsöffnung(en) und Austrittsfläche des Aerosolaustritts zugerechnet.

Die erfindungsgemäße Inhalationsvorrichtung ist so ausgelegt, dass aus der mindestens einen Düsenaustrittsöffnung austretendes Inhalat vor dem Auftreffen auf das Prallelement in Tröpfchen zerfällt (freier Strahlzerfall in Tropfen, insbesondere auch unabhängig von zusätzlichen Gasströmungen), und nicht erst ein kontinuierlicher Inhalatstrahl am Prallelement zerstäubt. Hierbei kann man sich den Strahlzerfall so vorstellen, dass sich aus einem aus der Inhalatdüse austretenden Flüssigkeitsstrahl ab einer gewissen Entfernung von der Düsenaustrittsöffnung eine geradlinige Töpfchenkette ausbildet.

Eine solche Auslegung kann empirisch durch einfache Auslegungsversuche erfolgen. Orientieren kann sich der Fachmann dabei an der folgenden Beziehung

$$Z = D \ln \frac{D}{2C} \sqrt{We}[1 + 3Oh]$$

für die Strahlaufbruchlänge Z, worin

$$We = \frac{\rho U^2 D}{\sigma}$$ die Weberzahl und $$Oh = \frac{\eta}{\sqrt{D\sigma\rho}}$$ die Ohnesorgezahl bezeichnet

mit

Z     Strahlaufbruchlänge in m
D     engster Durchmesser der Düsenaustrittsöffnung in m
C     Anfangsstörung des Strahlzerfalls in m
$\rho$     Dichte der physiologisch wirksamen Flüssigkeit in $kg/m^3$
$\sigma$     Oberflächenspannung der physiologisch wirksamen Flüssigkeit in N/m
$\eta$     Viskosität der physiologisch wirksamen Flüssigkeit in Pa s
U     die Austrittsgeschwindigkeit des Flüssigkeitsstrahls aus der Inhalatdüse

Die Anfangsstörung des Strahlzerfalls C ist in der Regel eine Unbekannte, für die vorliegende Erfindung hat sich jedoch erwiesen, dass für den dimensionslose Faktor

$$\ln \frac{2C}{D}$$

üblicherweise ein Wert zwischen 10 und 15, zumeist von 12 bis 13 angenommen werden kann.

Bei der Prallzerstäubung von durch freien Strahlenzerfall entstehende Tröpfchen in einer erfindungsgemäßen Vorrichtung sind gemäß Versuchswerten beispielsweise

Bei einem Düsendurchmesser von D = 20 $\mu$m und Drücken von 15 bis 25 bar:

$D_{v90}\approx$ 10 bis 13 $\mu$m
$D_{v50}\approx$ 6 bis 8 $\mu$m
$D_{v10}\approx$ 2,5 bis 4 $\mu$m

Bei einem Düsendurchmesser von D = 15 $\mu$m und Drücken von 15 bis 25 bar:

$D_{v90}\approx$ 8 bis 10 $\mu$m
$D_{v50}\approx$ 5 bis 7 $\mu$m
$D_{v10}\approx$ 2,5 bis 4 $\mu$m

Die Durchmesserangaben in den obigen Beispielen sind dabei wie folgt zu verstehen:

| | |
|---|---|
| $D_{v10}$ | 10% des Flüssigkeitsvolumens des Aerosols besteht aus Tröpfchen kleiner als $D_{v10}$ |
| $D_{v50}$ | 50% des Flüssigkeitsvolumens des Aerosols besteht aus Tröpfchen kleiner als $D_{v50}$ |
| $D_{v90}$ | 90% des Flüssigkeitsvolumens des Aerosols besteht aus Tröpfchen kleiner als $D_{v90}$ |

Physikalisch ist der Vorgang einer Prallzerstäubung von durch freien Strahlenzerfall entstehender Tröpfchen wie folgt zu verstehen: Der Zerstäubungsmechanismus gleicht dabei weniger dem makroskopischen Vorgang eines einzigen großen Tropfens, der auf ein Hindernis aufschlägt, sondern ist am besten zu beschreiben, indem man zwei nacheinander auf dieselbe Stelle auftreffende Tröpfchen betrachtet. Ein aufprallendes Tröpfchen bildet einen Film am Prallelement, in den ein nachfolgendes Tröpfchen einschlägt und eine "Krone" bildet, aus der dann kleinere Tröpfchen ablösen. In den Film zurückbleibender Restflüssigkeit kann dann wiederum ein nachfolgendes, aus freiem Strahlzerfall entstandenes Tröpfchen einschlagen und eine neue Krone bilden, aus der erneut kleiner Tröpfchen ablösen, usw.

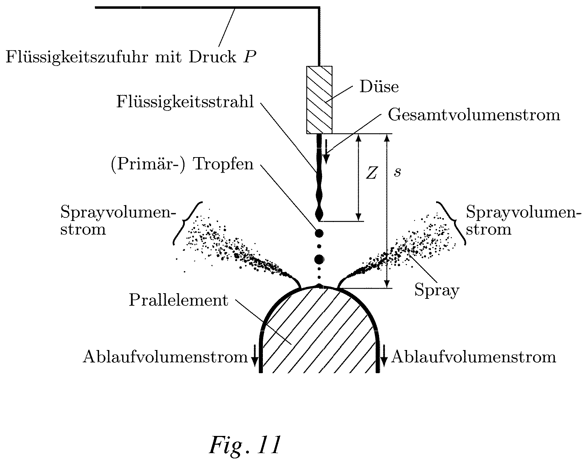

In Fig. 11 ist der Vorgang einer Prallzerstäubung von durch freien Strahlenzerfall entstehender Tröpfchen weiter veranschaulicht. Aus der Düse tritt ein Flüssigkeitsstrahl der Flüssigkeit aus, die der Düse unter Druck zugeführt wird. Nach der Strahlaufbruchlänge Z bricht der Flüssigkeitsstrahl in Primärtropfen auf, die nacheinander an (ungefähr) demselben Ort auf dem Prallelement auftreffen, das im Abstand s, der größer als die Strahlaufbruchlänge Z ist, gegenüber der Düsenöffnung angeordnet ist. Nach dem oben beschriebenen Vorgang sorgt jeder neu auftreffende Primärtropfen dafür, dass aus dem auf dem Prallelement befindlichen Flüssigkeitsfilm Sekundärtröpfchen ablösen, die einen Sprayvolumenstrom ausbilden. Ein Teil der Flüssigkeit läuft am Prallelement ab. Eine effiziente Prallzerstäubung wird also nicht dadurch erreicht, dass ein breiter Aerosolstrahl oder Sprühkegel auf eine Fläche aufgesprüht wird, so dass nur zufällig vereinzelte Tropfen auf einer Stelle auftreffen, auf der jeweils kurz zuvor bereits ein Tropfen aufgeprallt ist, vielmehr beruht das Konzept einer effektiven Prallzerstäubung darauf, dass ein, insbesondere durch freien Strahlenzerfall entstehender, Strom dicht aufeinanderfolgender Tropfen auf (näherungsweise) derselben Stelle auftrifft.

Im Zusammenhang mit der vorliegenden Erfindung wird das Inhalat als Flüssigkeit oder Suspension vorgelegt und der Inhalatdüse auch als Flüssigkeit oder Suspension zugeführt, d.h. die Inhalatdüse wird nicht mit einem Aerosol oder einer Flüssigkeit bzw. Suspension mit jeweils darin gelöstem Treibgas beschickt. Wenn eine Suspension als Inhalat vorgelegt wird, so sorgt eine derartige Ausgestaltung dafür, dass eine statische Aufladung der suspendierten Partikeln vermindert oder vermieden wird. Die suspendierten Partikeln verblieben in den Tröpfchen aus Suspensionsflüssigkeit ohne in größerem Ausmaß an den inneren Wandungen der Aerosolkammer abgeschieden zu werden.

Die Erfindung wird nachfolgend in beispielhafter Weise anhand der beigefügten schematischen Zeichnungen näher erläutert. Die Zeichnungen sind nicht maßstabsgetreu; insbesondere entsprechen Verhältnisse der einzelnen Abmessungen zueinander aus Gründen der Anschaulichkeit teilweise nicht den Abmessungsverhältnissen in tatsächlichen technischen Umsetzungen. Es werden mehrere bevorzugte Ausführungsbeispiele beschrieben, auf welche die Erfindung jedoch nicht beschränkt ist.

Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

Es zeigt

Fig. 1    im Querschnitt eine Inhalationsvorrichtung mit Druckbehälter als Inhalatvorlage und einem Inhalationsaktuator mit einem Saugvlies zur Aufnahme vom Prallelement abtropfenden Inhalats,

Fig. 2    im Querschnitt eine Inhalationsvorrichtung wie in Fig. 1, wobei im Unterschied hierzu die Düsenaustrittsöffnung j edoch in die zum Aerosolaustritt entgegengesetzte Richtung weist,

Fig. 3    im Querschnitt eine Inhalationsvorrichtung mit Druckbehälter als Inhalatvorlage und einem Inhalationsaktuator, dessen Aerosolkammer eine Luftzufuhr mit Flatterventil aufweist,

Fig. 4    im Querschnitt eine Inhalationsvorrichtung ähnlich Fig. 3 mit von der Aerosolkammer abgegrenztem Mundstück und gegenüber Fig. 3 veränderter Anordnung des Prallelements,

Fig. 5    im Querschnitt eine erfreze--e Inhalationsvorrichtung wie in Fig. 4, wobei anstelle eines Saugvlieses eine Auffangkammer zur Aufnahme vom Prallelement abtropfenden Inhalats vorgesehen ist und auf ein Flatterventil am Aerosolaustritt verzichtet wird,

Fig. 6    im Querschnitt eine einfache Ausführung einer Inhalationsvorrichtung ohne Flatterventil und mit Auffangkammer zur Aufnahme vom Prallelement abtropfenden Inhalats,

Fig. 7    im Querschnitt eine Inhalationsvorrichtung wie in Fig. 2, wobei im Unterschied hierzu das Prallelement als Vorsprung in der Wandung der Aerosolkammer ausgebildet ist,

Fig. 8    im Querschnitt eine Inhalationsvorrichtung wie in Fig. 1, wobei im Unterschied hierzu die Inhalatdüse etwas zum Druckbehälter hin versetzt und das Prallelement als Vorsprung in der Wandung der Aerosolkammer ausgebildet ist,

Fig. 9a    im Querschnitt eine erfindungsgemäße Inhalationsvorrichtung, bei welcher das Prallelement auf einem Einsatz angeordnet ist, und die lokale Verengung des Aerosolaustritts auf einem weiteren Einsatz angeordnet ist,

Fig. 9b    den Einsatz aus Fig. 9a, auf dem das Prallelement angeordnet ist, in einer Frontalansicht (von rechts in Fig. 9a gesehen),

Fig. 9c    den Einsatz aus Fig. 9b in der Rückansicht,

Fig. 9d    den Einsatz aus Fig. 9a, auf dem die lokale Verengung des Aerosolaustritts angeordnet ist, in einer Ansicht von links in Fig. 9a,

Fig. 10a    im Querschnitt eine erfindungsgemäße Inhalationsvorrichtung ähnlich Fig. 9a, bei welcher allerdings das Prallelement und die lokale Verengung des Aerosolaustritts auf einem gemeinsamen Einsatz angeordnet sind,

Fig. 10b    den Einsatz aus Fig. 10a in einer Frontalansicht (von rechts in Fig. 10a gesehen),

Fig. 10c    den Einsatz aus Fig. 10b in der Rückansicht,

Fig. 10d    die Rückansicht eines alternativen Einsatzes ähnlich Fig. 10c,

Fig. 10e    eine Seitenansicht des alternativen Einsatzes aus Fig. 10d, entsprechend einer Ansicht von oben oder unten in Fig. 10f,

Fig. 10f    den alternativen Einsatz aus Figuren 10d und 10d in einer Querschnittsansicht entsprechend Fig. 10a, und

Fig. 11    eine Veranschaulichung des Vorgangs der Prallzerstäubung.

In den einzelnen Figuren sind einander entsprechende Elemente jeweils mit denselben Bezugzeichen gekennzeichnet.

Die Inhalatvorlage 1 ist ausgebildet wie bei einem herkömmlichen Dosierspray aus dem medizinischen Anwendungsbereich und weist einen Druckbehälter 2, eine Dosierventileinheit 3 und einen Austragsstutzen 4 auf. Inhalat kann durch den Austragsstutzen 4 aus dem Druckbehälter 2 austreten, wenn der Austragsstutzen 4 axial in die Dosierventileinheit 3 eingedrückt wird. Das Eindrücken des in die Aufnahme 5 des Inhalationsaktuators 6 eingesetzten Austragsstutzens 4 in die Dosierventileinheit 3 erfolgt, indem der Inhalationsaktuator 6 (nachfolgend auch als Aktuator 6 bezeichnet) und der Druckbehälter 2 zueinander gedrückt werden. Hierfür kann der Anwender die Hülle 7 des Aktuators 6 mit dem Handballen und den übrigen vier Fingern einer Hand greifen, während er mit dem Daumen den Druckbehälter 2 gegen die Federkraft einer in der Dosierventileinheit 3 vorgesehenen Rückstellfeder (nicht dargestellt) nach unten drückt und somit einen Sprühstoß auslöst. Nach Lösen des Daumendrucks wird der Druckbehälter 2 von der Rückstellfeder wieder in seine Ausgangslage zurückgestellt, und die Vorrichtung ist bereit zur Betätigung für einen weiteren Sprühstoß. Vorzugsweise wird jedoch die gesamte Anordnung zwischen Daumen und Zeigefinger gehalten und zusammengedrückt, wobei der Daumen unten am Aktuator 6 und der Zeigefinger oben am Boden des Druckbehälters 2 angesetzt wird. Zur Unterstützung kann hier vorteilhafterweise eine Daumenmulde 22 im Kopfbereich des Aktuators 6 vorgesehen sein.

Inhalat, das während eines Sprühstoßes austritt, gelangt durch die Inhalatzuführung 8 zur Inhalatdüse 9. Die Inhalatdüse 9 ist in den Zeichnungen als in die Aufnahme 5 eingesetzter, beispielsweise lasertechnisch gebohrter Düsenkörper dargestellt. Die Inhalatdüse kann aber auch einstückig mit der Aufnahme ausgebildet sein. Der vom Treibmitteldruck des Druckbehälters bewirkte Druckgradient treibt das flüssige Inhalat durch die Inhalatdüse 9, aus deren Düsenaustrittsöffnung es gegen das Prallelement 10 gesprüht wird.

Der Treibmitteldruck im Druckbehälter 2, der Durchmesser der Düsenaustrittsöffnung und der Abstand zwischen Düsenaustrittsöffnung und Prallelement 10 sind so aufeinander sowie auf die physikalischen Eigenschaften des Inhalats abgestimmt, dass die Inhalatflüssigkeit nach dem Austreten aus der Düsenaustrittsöffnung durch freien Strahlzerfall in Tröpfchen zerfällt, welche dann auf das Prallelement 10 auftreffen. Durch den Aufprall auf das Prallelement 10 werden die Inhalattröpfchen weiter zerstäubt und abgebremst. Die Tröpfchengrößenverteilung des entstehenden Aerosols ist gegenüber den durch freien Strahlzerfall entstandenen Tropfen zu niedrigeren Tröpfchendurchmessern hin verschoben. Somit lässt sich eine gute Lungengängigkeit für das Inhalat erzielen. Überschüssiges, vom Prallelement 10 abtropfendes Inhalat wird von dem saugfähigen Materialstück (Vlies) aufgenommen.

Dass das Prallelement 10 die direkte Verbindung zwischen Düsenaustrittsöffnung und Aerosolaustritt 11 unterbricht, sorgt zusammen mit dem Abbremsen der Inhalattröpfchen dafür, dass Inhalat nicht bereits aufgrund des Druckgradienten an der Inhalatdüse mit hoher Geschwindigkeit aus dem Aerosolaustritt 11 herausgeschleudert wird. Stattdessen kann sich das während des Sprühstoßes entstehende Aerosol im Innenvolumen der Aerosolkammer 12 sammeln, welche mit der Aufnahme einstückig ausgeführt ist.

Der Anwender kann das Aerosol aus der Aerosolkammer 12 durch den, gegenüber der Aerosolkammer 12 verengten, Aerosolaustritt 11 hindurch einatmen. Hierfür besitzt die Aerosolkammer 12 eine von der Inhalatdüse 9 und dem Aerosolaustritt 11 separate Luftzufuhr 13, welche in der abgebildeten Ausführungsform dadurch gebildet ist, dass die den Spalt zwischen Druckbehälter 2 und Hülle 7 des Aktuators 6 angeordnete Dichtung porös ausgeführt ist.

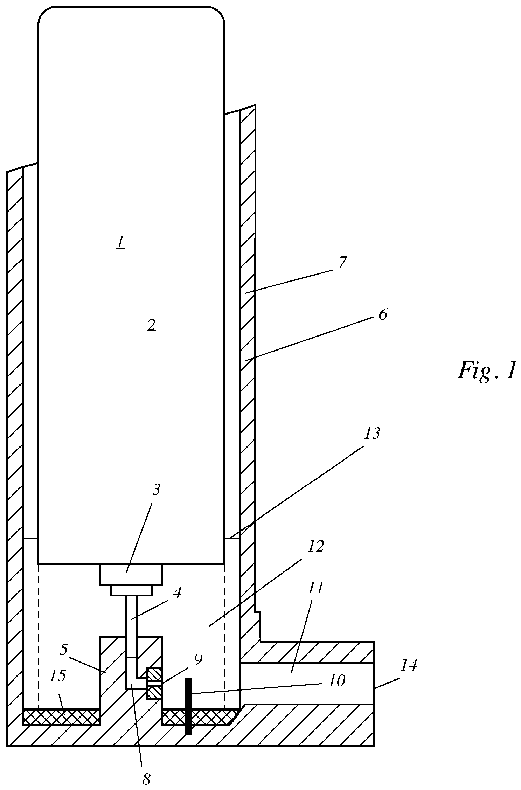

Der Großteil, d.h. mehr als 50% des Innenvolumens der Aerosolkammer 12 liegt innerhalb der, in Fig. 1 mittels unterbrochenen Linien angedeuteten, Projektion des Druckbehälters 2 in Richtung seiner Längsachse, zumindest wenn die Rückstellfeder in der Dosierventileinheit 3 entspannt ist, und die Aerosolkammer 12 ihre maximale Ausdehnung hat. Zum Innenvolumen der Aerosolkammer zählt das durchströmbare Volumen im Aerosolaustritt 11 bis zur Austrittsfläche 14. Die Austrittsfläche 14 ist die am weitesten anwenderseitig angeordnete Fläche mit dem geringsten durchströmbaren Flächeninhalt des Aerosolaustritts 11. Da im vorliegenden Ausführungsbeispiel die durchströmbare Fläche des röhrenförmigen Aerosolaustritts 11 über einen größeren Bereich konstant ist, ist die Austrittsfläche 14 im Bild ganz rechts gelegen.

Mehr als 25 Prozent des Innenvolumens der Aerosolkammer 12 sind, bezogen auf die Richtung, die durch eine gerade Verbindungslinie von der Düsenaustrittsöffnung der Inhalatdüse 9 zum Flächenschwerpunkt der Austrittsfläche 14 des Aerosolaustritts 11 definiert ist, hinter der Düsenaustrittsöffnung angeordnet, und zwar wenn die Rückstellfeder in der Dosierventileinheit 3 entspannt ist, aber auch noch wenn Aktuator 6 und Druckbehälter 2 maximal zueinander gedrückt sind.

Im Vergleich zu herkömmlichen Spacern ist die Aerosolkammer 12 überaus kompakt ausgeführt, so dass sie in den Aktuator 6 integriert sein kann, und sich dessen maximale Ausdehnung dennoch parallel zum in die Aufnahme 5 eingeführten Austragsstutzen 4, d.h. die maximale Ausdehnung der Inhalationsvorrichtung in Richtung der Längsachse des Druckbehälters 2 erstreckt.

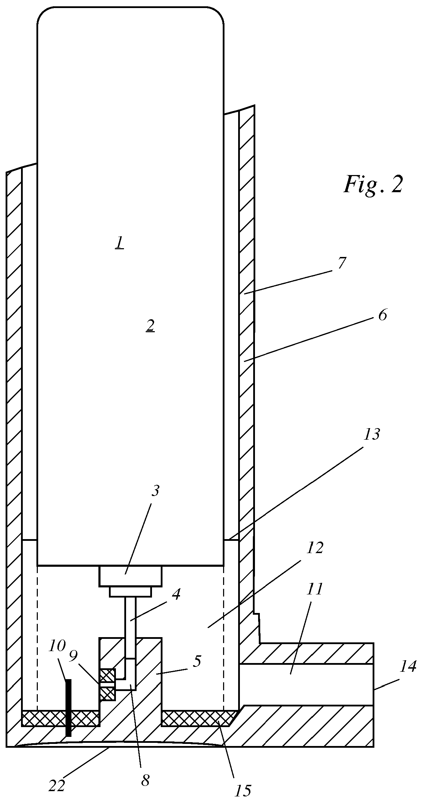

Die Inhalationseinrichtung in Fig. 2 ist im wesentlichen wie die Inhalationsvorrichtung aus Fig. 1 ausgebildet. Allerdings ist die Hauptaustragsrichtung der Düsenaustrittsöffnung der Inhalatdüse 9 in einem Winkel 180° zur Hauptaustragsrichtung des Aerosolaustritts 11 angeordnet. Die Hauptaustragsrichtung des Aerosolaustritts 11 steht orthogonal auf dem Flächenschwerpunkt der Austrittsfläche 14. Die Hauptaustragsrichtung der Düsenaustrittsöffnung steht orthogonal auf dem Flächenschwerpunkt der kleinsten durchströmbaren Fläche der Düsenaustrittsöffnung.

Mehr als 10 Prozent des Innenvolumens der Aerosolkammer 12 sind, bezogen auf die Richtung, die durch eine gerade Verbindungslinie von der Düsenaustrittsöffnung der Inhalatdüse 9 zum Flächenschwerpunkt der Austrittsfläche 14 des Aerosolaustritts 11 definiert ist, hinter der Düsenaustrittsöffnung angeordnet, und zwar wenn die Rückstellfeder in der Dosierventileinheit 3 entspannt ist, aber auch noch wenn Aktuator 6 und Druckbehälter 2 maximal zueinander gedrückt sind.

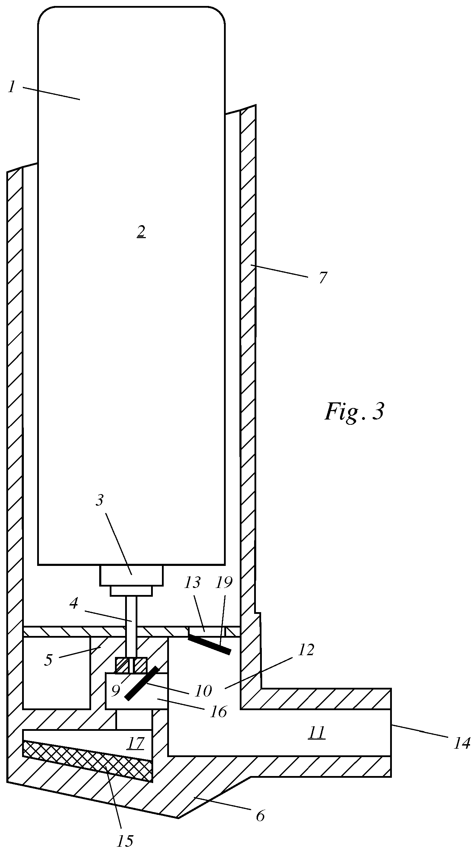

Die Inhalationseinrichtung in Fig. 3 weist ebenfalls eine Inhalatvorlage 1 mit Druckbehälter 2, Dosierventileinheit 3 und Austragsstutzen 4 auf. Die Inhalatzuführung in der Aufnahme 5 des Aktuators 6 ist sehr kurz ausgeführt; die Inhalatdüse 9 sitzt praktisch direkt am der Dosierventileinheit 3 gegenüberliegenden Ende des Austragsstutzens 4, so dass die Düsenaustragsöffnung in etwa koaxial zum Austragsstutzen 4 angeordnet ist. Von der Düsenaustragsöffnung gelangt das Inhalat in eine kleine, mit der Aerosolkammer 12 in fluidischer Verbindung stehende Vorkammer 16, in der das Prallelement 10 angeordnet ist. Die Oberfläche des Prallelements 10 steht in einem Winkel von etwa 45° zur Hauptaustragsrichtung der Düsenaustrittssöffnung zwischen der Düsenaustrittssöffnung und der fluidischen Verbindung der Vorkammer 16 zur Aerosolkammer 12. Diese Anordnung dient der weitergehenden Abbremsung des Inhalats.

Unterhalb der Vorkammer 16 und mit dieser in fluidischer Verbindung stehend ist die Inhalatauffangkammer 17 angeordnet, welche saugfähiges Material 15, beispielsweise aus Vlies oder Silikat, enthält.

Gegenüber der Aerosolkammer 12 verengt ist wiederum der Aerosolaustritt 11, durch welchen zerstäubtes Inhalat aus der Aerosolkammer 12 inhaliert werden kann. Der röhrenförmige Aerosolaustritt 11 kann dabei auch als von den Lippen des Anwenders umschließbares Mundstück dienen oder aber als Adapter für den Anschluss einer Maske oder dergleichen.

Die von der Inhalatdüse 9 und dem Aerosolaustritt 11 separate Luftzufuhr 13 ist mit einem Klappenventil bzw. Flatterventil 19 ausgestattet.

Der Großteil, d.h. mehr als 50% des Innenvolumens der Aerosolkammer 12 liegt wieder innerhalb der Projektion des Druckbehälters 2 in Richtung seiner Längsachse. Zum Innenvolumen der Aerosolkammer zählt das durchströmbare Volumen im Aerosolaustritt 11 bis zur Austrittsfläche 14.

Auch hier ist die Aerosolkammer 12 im Vergleich zu herkömmlichen Spacern überaus kompakt ausgebildet, so dass sie in den Aktuator 6 integriert sein kann, und sich dessen maximale Ausdehnung dennoch parallel zum in die Aufnahme 5 eingeführten Austragsstutzen 4, d.h. die maximale Ausdehnung der Inhalationsvorrichtung in Richtung der Längsachse des Druckbehälters 2 erstreckt.

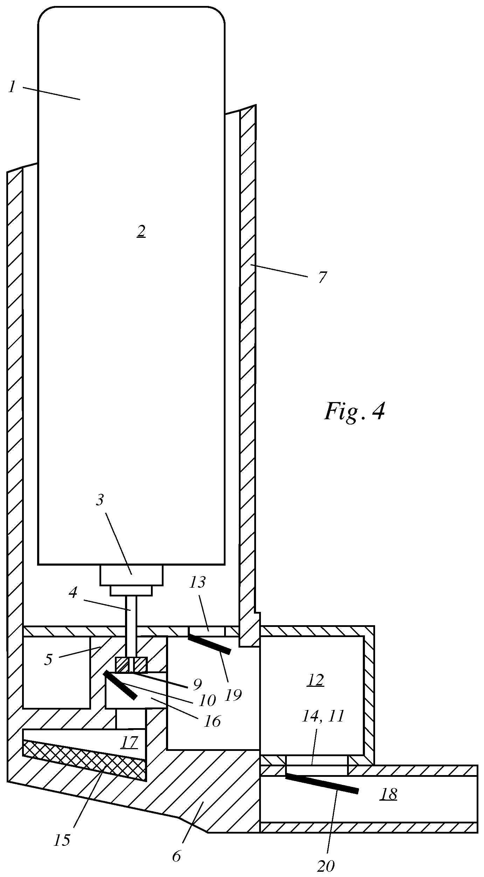

Die Inhalationsvorrichtung in Fig. 4 ist ähnlich ausgeführt wie in Fig. 3. Sie weist ebenfalls eine Inhalatvorlage 1 mit Druckbehälter 2, Dosierventileinheit 3 und Austragsstutzen 4 auf. Die Dosierventileinheit 3 ist wiederum bedienbar indem entweder die Hülle 7 gegriffen und der Druckbehälter 2 mit dem Daumen nach unten gedrückt wird, oder aber vorzugsweise die gesamte Anordnung zwischen Daumen und Zeigefinger gehalten und zusammengedrückt wird, wobei der Daumen unten am Aktuator und der Zeigefinger oben am Boden des Druckbehälters 2 angesetzt wird. Zur Unterstützung kann hier vorteilhafterweise eine Daumenmulde (nicht dargestellt) im Kopfbereich des Aktuators 6 vorgesehen sein.

Die Inhalatzuführung 8 in der Aufnahme 5 des Aktuators 6 fällt mit dem Einlass der Inhalatdüse 9 zusammen, die direkt am der Dosierventileinheit 3 gegenüberliegenden Ende des Austragsstutzens 4 sitzt, so dass die Düsenaustragsöffnung in etwa koaxial zum Austragsstutzen 4 angeordnet ist. Von der Düsenaustragsöffnung gelangt das Inhalat in eine kleine, mit der Aerosolkammer 12 in fluidischer Verbindung stehende Vorkammer 16, in der das Prallelement 10 angeordnet ist. Die Oberfläche des Prallelements 10 steht in einem Winkel von etwa 45° zur Hauptaustragsrichtung der Düsenaustrittssöffnung wie auch zur Öffnung der Verbindung der Vorkammer 16 in die Aerosolkammer 12.

Unterhalb der Vorkammer 16 und mit dieser in fluidischer Verbindung stehend ist die Inhalatauffangkammer 17 angeordnet, welche saugfähiges Material 15, beispielsweise aus Vlies oder Silikat, enthält.

Gegenüber der Aerosolkammer 12 verengt ist wiederum der Aerosolaustritt 11, durch welchen zerstäubtes Inhalat aus der Aerosolkammer 12 gelangen und weiter durch das Mundstück 18 inhaliert werden kann. Die Hauptaustragsrichtung des Aerosolaustritts 11 ist in etwa parallel zur Hauptaustragsrichtung der Düsenaustrittsöffnung, jedoch senkrecht zur Hauptaustragsöffnung der Verbindungsöffnung zwischen Vorkammer 16 und Aerosolkammer 12 angeordnet.

Die von der Inhalatdüse 9 und dem Aerosolaustritt 11 separate Luftzufuhr 13 ist mit einem Klappenventil bzw. Flatterventil 19 ausgestattet.

Ebenfalls mit einem Flatterventil 20 ausgestattet ist der Aerosolaustritt 11.

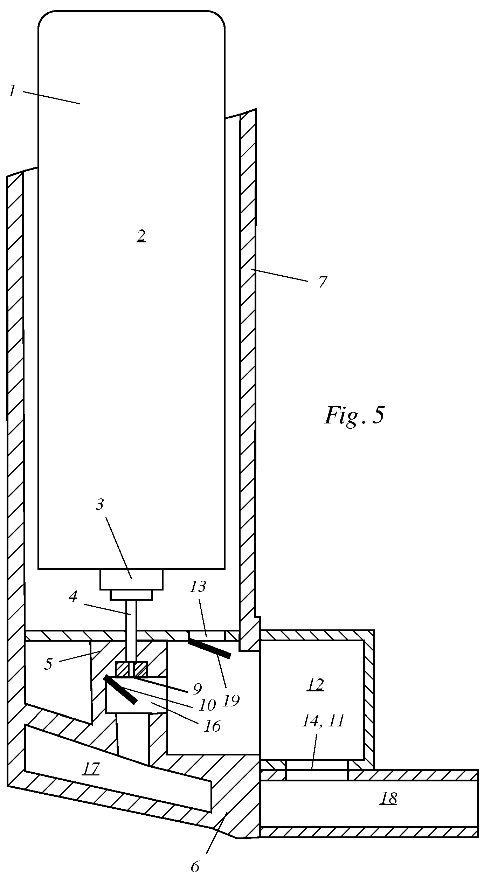

Die Inhalationsvorrichtung in Fig. 5 ist weitgehend gleich ausgeführt wie in Fig. 4. Anstelle der Inhalatauffangkammer in Fig. 4, welche saugfähiges Material enthält, ist die Inhalatauffangkammer 17 jedoch größer und ohne saugfähiges Material gestaltet.

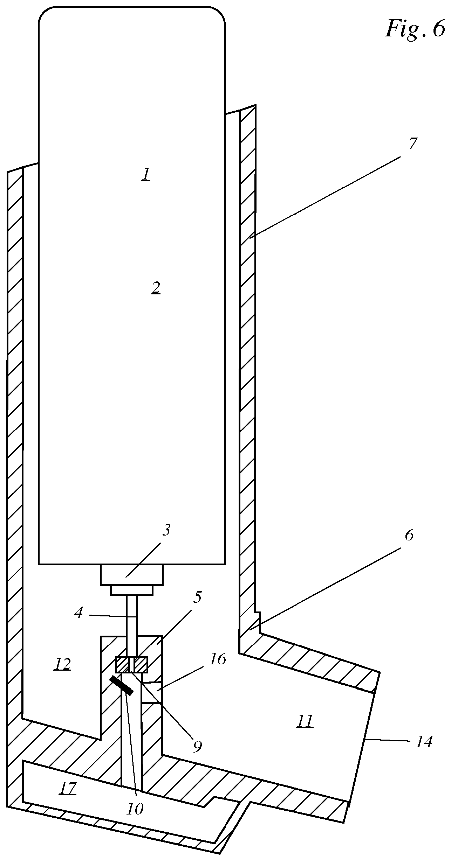

Auch die besonders einfach ausgeführte Inhalationsvorrichtung in Fig. 6 besitzt eine Inhalatauffangkammer 17 ohne saugfähiges Material.

Die Inhalatvorlage 1 weist den Druckbehälter 2, die Dosierventileinheit 3 und den Austragsstutzen 4 auf. Die Dosierventileinheit 3 ist wiederum bedienbar indem die Hülle 7 gegriffen und der Druckbehälter 2 mit dem Daumen nach unten gegen den Aktuator 6 gedrückt wird, oder aber vorzugsweise die gesamte Anordnung zwischen Daumen und Zeigefinger gehalten und zusammengedrückt wird, wobei der Daumen unten am Aktuator und der Zeigefinger oben am Boden des Druckbehälters 2 angesetzt wird. Zur Unterstützung kann hier vorteilhafterweise eine Daumenmulde im Kopfbereich des Aktuators 6 vorgesehen sein.

Die Inhalatzuführung 8 in der Aufnahme 5 des Aktuators 6 fällt mit dem Einlass der Inhalatdüse 9 zusammen, die direkt am der Dosierventileinheit 3 gegenüberliegenden Ende des Austragsstutzens 4 sitzt, so dass die Düsenaustragsöffnung in etwa koaxial zum Austragsstutzen 4 angeordnet ist.

Wie in Fig. 5 gelangt das Inhalat von der Düsenaustragsöffnung in eine kleine, mit der Aerosolkammer 12 in fluidischer Verbindung stehende Vorkammer 16, in der das Prallelement 10 angeordnet ist. Die Oberfläche des Prallelements 10 steht in einem Winkel von etwa 45° zur Hauptaustragsrichtung der Düsenaustrittssöffnung wie auch zur Öffnung der Verbindung der Vorkammer 16 in die Aerosolkammer 12.

Der Lufteinlass 13 in die Aerosolkammer 12 wird durch den Ringspalt zwischen Aktuatorhülle 7 und Druckbehälter 2 gebildet. Das in der Darstellung untere Ende des Druckbehälters 2 fungiert zugleich als in der Darstellung obere Wandung der Aerosolkammer 12.

Gegenüber der Aerosolkammer 12 verengt ist wiederum der Aerosolaustritt 11, durch welchen zerstäubtes Inhalat aus der Aerosolkammer 12 inhaliert werden kann. Der Aerosolaustritt 11 dient dabei wiederum als Mundstück, das der Anwender mit den Lippen umschließen kann.

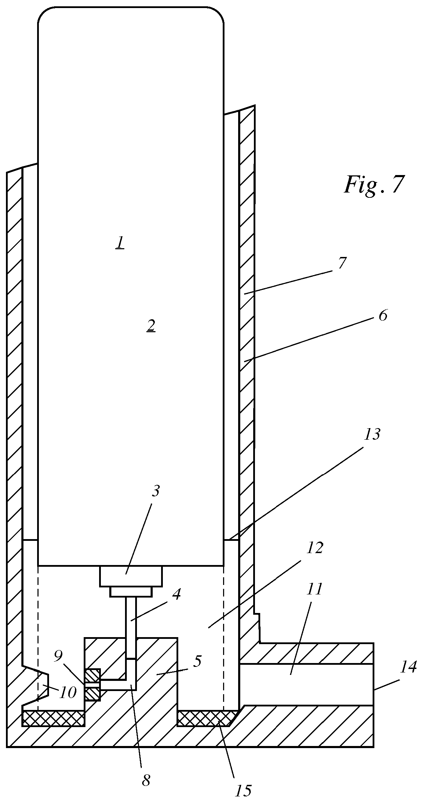

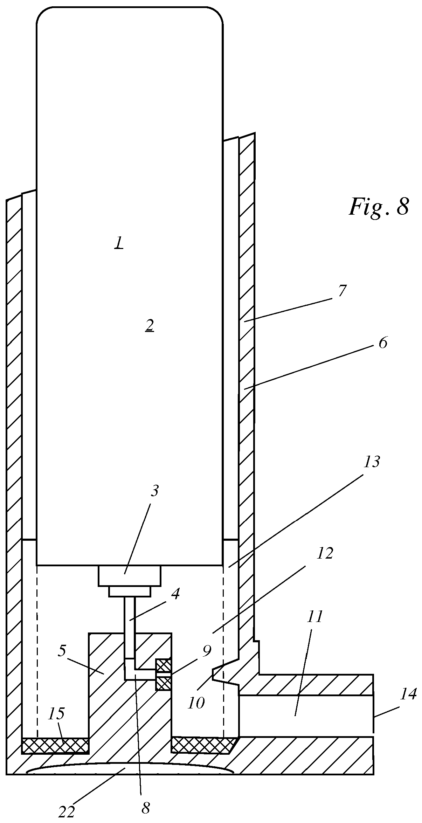

Während das Prallelement 10 in Figuren 1-6 als separates Bauteil, beispielsweise aus einem Kunststoff, metallischen oder keramischen Werkstoff, gefertigt und in den Aktuator 6 eingesetzt ist, kann das Prallelement 10 auch integral ausgebildet werden. Entsprechend zeigt Fig. 7 eine Fig. 2 weitestgehend entsprechende Inhalationsvorrichtung, wobei jedoch das Prallelement 10 als Ausformung der Wandung der Aerosolkammer 12 ausgebildet ist. Die Inhalationsvorrichtung in Fig. 8 entspricht weitgehend der Inhalationsvorrichtung aus Fig. 1, allerdings ist hier ebenfalls das Prallelement 10 als Ausformung der Wandung der Aerosolkammer 12 ausgebildet.

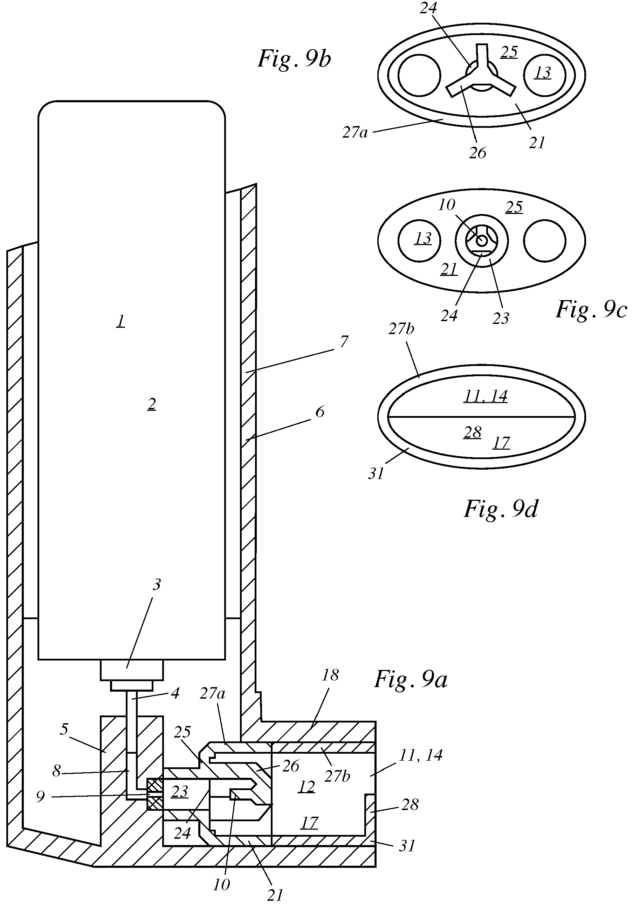

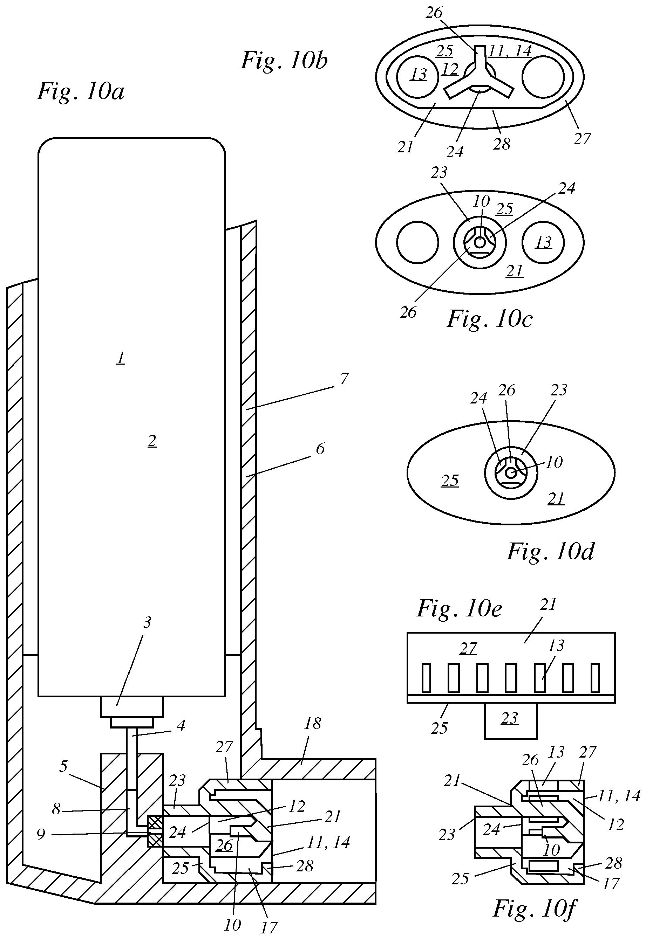

Figuren 9a und 10a zeigen wiederum Ausführungen im Querschnitt, bei denen das Prallelement 10 separat von der Aufnahme 5 und Hülle 7 ausgeführt ist. In diesen Ausführungsbeispielen ist das Prallelement in den Einsatz 21 integriert, der - in Figuren 9a und 10a jeweils von rechts - in das Mundstück 18 eingeschoben ist. Einschieberichtung entsprechend obigen Definitionen ist in Figuren 9a und 10a also jeweils von rechts nach links.

Der Einsatz 21 aus Fig. 9a ist in Fig. 9b ohne den restlichen Inhalationsaktuator in der Draufsicht in Einschieberichtung (also in Fig. 9a von rechts) dargestellt, in Fig. 9c in entsprechender Rückansicht, also mit Blickrichtung entgegen der Einschieberichtung (in Fig. 9a von links). Gleichermaßen ist der Einsatz 21 aus Fig. 10a in Fig. 10b ohne den restlichen Inhalationsaktuator in der Draufsicht in Einschieberichtung (also in Fig. 10a von rechts) dargestellt, in Fig. 10c in entsprechender Rückansicht, also mit Blickrichtung entgegen der Einschieberichtung (in Fig. 10a von links).

Fig. 10d zeigt in der zu Fig. 10c analogen Rückansicht einen alternativen Einsatz 21. In Fig. 10e ist dieser alternative Einsatz 21 in einer seitlichen Ansicht abgebildet, d.h. von unten (oder von oben - was aufgrund der Symmetrie keinen Unterschied ausmacht) in Figuren 10d und 10f. Die Querschnittansicht des alternativen Einsatzes 21 in Fig. 10f ist analog der Querschnittsansicht in Fig. 10a abgebildet.

Über den Stutzen 23 kann das flüssige Inhalat von der Inhalatdüse 9, aus deren Düsenaustrittsöffnung es austritt, durch die Inhalateintrittsöffnung 24 in der quer zur Einschiebrichtung angeordneten Wandung 25 des Einschubs 21 gegen das Prallelement 10 gesprüht werden. Das Prallelement 10 wird von den in den Einsatz 21 integrierten Streben 26 gehalten. Die Wandung 25 stellt die Rückwand der Aerosolkammer 12 dar.

Lateral wird die Aerosolkammer 12 von der ovalen Röhre 27a (in Fig. 9a, 9b) bzw. 27 (in Fig. 10a, 10b) begrenzt, die zugleich als Zentriermittel zum Zentrieren des Einsatzes 21 im Mundstück 18 dient. In Fig. 9a ist die ovale Röhre 27a entgegen der Einschieberichtung über die ovale Röhre 27b des weiteren Einsatzes 31 verlängert, der ebenfalls in das Mundstück 18 eingeschoben ist. Die Röhre 27b dient wiederum als Zentriermittel zum Zentrieren des weiteren Einsatzes 31 im Mundstück 18.

Der weitere Einsatz 31 ist in Fig. 9d mit Blickrichtung entgegen der Einschieberichtung (in Fig. 9a von links) dargestellt. Zusammen mit dem Einsatz 21 bildet er die Aerosolkammer 12 aus.

Der asymmetrische Rand 28 verengt lokal den Aerosolaustritt 11 aus der Aerosolkammer 12, d.h. er begrenzt dessen Austrittsfläche 14. Wenn der Inhalationsaktuator, wie in Figuren 9a und 10a dargestellt, bestimmungsgemäß über Kopf gehalten wird, dann ist mittels des Randes 28 und der Wandung 25 auch eine Inhalatauffangkammer 17 gebildet, die verhindert, dass vom Prallelement 10 abtropfendes überschüssiges Inhalat aus der Öffnung des Mundstücks 18 rinnt.

Durch den Lufteinlass 13 kann Luft in die Aerosolkammer 12 einströmen. Im Einsatz 21 der Figuren 9b, 9c, 10b und 10c ist der Lufteinlass 13 in Form von Öffnungen in der Wandung 25, also der Rückwand der Aerosolkammer 12 gebildet. Im alternativen Einsatz 21 der Figuren 10d, 10e besteht der Lufteinlass 13 aus Wandunterbrechungen in einem Abschnitt der Röhre 27, der nicht vollständig von der Wandung des Mundstücks 18 überdeckt ist.

Die Inhalationsvorrichtungen in Figuren 9a und 10a weisen ebenfalls eine Inhalatvorlage 1 mit Druckbehälter 2, Dosierventileinheit 3 und Austragsstutzen 4 auf. Die Dosierventileinheit 3 ist wiederum bedienbar indem entweder die Hülle 7 gegriffen und der Druckbehälter 2 mit dem Daumen nach unten gedrückt wird, oder aber vorzugsweise die gesamte Anordnung zwischen Daumen und Zeigefinger gehalten und zusammengedrückt wird, wobei der Daumen unten am Aktuator und der Zeigefinger oben am Boden des Druckbehälters 2 angesetzt wird. Zur Unterstützung kann hier vorteilhafterweise wiederum eine Daumenmulde (nicht dargestellt) im Kopfbereich des Aktuators 6 vorgesehen sein.

Der Treibmitteldruck im Druckbehälter 2, der Durchmesser der Düsenaustrittsöffnung und der Abstand zwischen Düsenaustrittsöffnung und Prallelement 10 sind wiederum so aufeinander sowie auf die physikalischen Eigenschaften des Inhalats abgestimmt, dass die Inhalatflüssigkeit nach dem Austreten aus der Düsenaustrittsöffnung durch freien Strahlzerfall in Tröpfchen zerfällt, welche dann auf das Prallelement 10 auftreffen. Durch den Aufprall auf das Prallelement 10 werden die Inhalattröpfchen weiter zerstäubt und abgebremst.

**Patentansprüche**

1.  Inhalationsvorrichtung, aufweisend

    eine Inhalatvorlage (1) und
    einen Inhalationsaktuator (6), der folgendes aufweist:

      eine Aufnahme (5) zum Aufnehmen eines Austragsstutzens (4) der Inhalatvorlage (1),
      eine Inhalatdüse (9) zum Sprühen von Inhalat aus mindestens einer Düsenaustrittsöffnung der Inhalatdüse (9),
      eine Inhalatzuführung (8) zum Zuführen von Inhalat vom Austragsstutzen (4) zur Inhalatdüse (9),
      ein Prallelement (10), auf welches Inhalat aus der Düsenaustrittsöffnung zur Prallzerstäubung geradlinig aufsprühbar ist,
      eine Aerosolkammer (12) mit einem Innenvolumen, dem aus der Inhalatdüse (9) gesprühtes Inhalat zuführbar ist,

    wobei die Aerosolkammer (12) einen Aerosolaustritt (11) zum Ausströmen von Aerosol aus der Aerosolkammer (12) und eine von der Inhalatdüse (9) und dem Aerosolaustritt (11) separate Luftzufuhr (13) aufweist, und wobei die Inhalationsvorrichtung so ausgelegt ist, dass aus der mindestens einen Düsenaustrittsöffnung als Flüssigkeitsstrahl austretendes Inhalat vor dem Auftreffen auf das Prallelement (10) durch freien Zerfall in Tröpfchen zerfällt, und dass die Tröpfchen dann zur Prallzerstäubung auf das Prallelement (10) auftreffen, **dadurch gekennzeichnet, dass** der Aerosolaustritt (11) eine lokale Verengung aufweist.

2.  Inhalationsvorrichtung gemäß Anspruch 1,
    aufweisend einen Einsatz (21), der mindestens eine Wandung der Aerosolkammer (12) bildet und das Prallelement (10) trägt.

3.  Inhalationsvorrichtung gemäß einem der vorangehenden Ansprüche, wobei die Aufnahme (5), die Inhalatzuführung (8) und die Aerosolkammer (12) gemeinsam einstückig ausgeführt sind.

4.  Inhalationsvorrichtung gemäß einem der vorangehenden Ansprüche, wobei die mindestens eine Düsenaustrittsöffnung und der Aerosolaustritt (11) relativ zueinander so angeordnet sind, dass Inhalat nicht geradlinig vom Düsenaustritt zum Aerosolaustritt (11) strömen kann.

5.  Inhalationsvorrichtung gemäß einem der vorangehenden Ansprüche, wobei die Hauptaustragsrichtung der mindestens einen Düsenaustrittsöffnung und die Hauptaustragsrichtung des Aerosolaustritts (11) versetzt zueinander angeordnet sind und/oder in einem Winkel größer null und kleiner 180° zueinander stehen, wobei die Hauptaustragsrichtung als Senkrechte zum Flächenschwerpunkt der jeweils kleinsten durchströmbaren Fläche der Düsenaustrittsöffnung bzw. des Aerosolaustritts (11) definiert ist.

6.  Inhalationsvorrichtung gemäß Anspruch 5, wobei die Hauptaustragsrichtung der mindestens einen Düsenaustrittsöffnung und die Hauptaustragsrichtung des Aerosolaustritts (11) in einem Winkel größer 29° und kleiner 151° zueinander stehen.

7.  Inhalationsvorrichtung gemäß einem der vorangehenden Ansprüche, wobei die Luftzufuhr (13) mit einem Luftzufuhrventil (19) ausgestattet ist.

8.  Inhalationsvorrichtung gemäß einem der vorangehenden Ansprüche, wobei sich die maximale Ausdehnung des Inhalationsaktuators (2) parallel zum bestimmungsgemäß in die Aufnahme (5) eingeführten Austragsstutzen (4) erstreckt.

9. Inhalationsvorrichtung gemäß einem der vorangehenden Ansprüche, wobei zumindest 10 Prozent, vorzugsweise zumindest 25 Prozent des Innenvolumens der Aerosolkammer (12) bezogen auf eine Richtung, die durch eine gerade Verbindungslinie von der Düsenaustrittsöffnung zum Flächenschwerpunkt der Austrittsfläche des Aerosolaustritts (11) definiert ist, hinter der Düsenaustrittsöffnung angeordnet sind.

10. Inhalationsvorrichtung gemäß Anspruch 2, wobei der Inhalationsaktuator

ein vor der mindestens einen Düsenaustrittsöffnung angeordnetes Mundstück (18) aufweist, und
wobei der Einsatz (21) folgendes aufweist:

Zentriermittel zum Zentrieren des Einsatzes (21) im Mundstück (18),
eine quer zur Einschieberichtung angeordnete Wandung (25), welche in Einschieberichtung vor den Zentriermitteln angeordnet ist,
eine in der Wandung (25) vorgesehene Inhalateintrittsöffnung (24),
das Prallelement (10), welches entgegen der Einschieberichtung vor der Inhalateintrittsöffnung (24) angeordnet ist,
eine quer zur Einschieberichtung neben dem Prallelement (10) angeordnete, in Einschieberichtung frei durchströmbare Fläche,
den in Einschieberichtung vor der frei durchströmbaren Fläche angeordneten Lufteinlass (13), und
den entgegen der Einschieberichtung vor der frei durchströmbaren Fläche angeordneten Aerosolaustritt (11),
wobei der Einsatz (21) so in das Mundstück (18) eingeschoben ist, dass ein geradliniger Strömungspfad von der mindestens einen Düsenaustrittsöffnung durch die Inhalateintrittsöffnung (24) zum Prallelement (10) besteht.

11. Inhalationsvorrichtung gemäß Anspruch 10,
wobei die Zentriermittel integral mit einer durchgehenden oder unterbrochenen Röhre (27a) ausgeführt sind, wobei die Längsrichtung der Röhre (27a) der Einschieberichtung entspricht.

12. Inhalationsvorrichtung gemäß Anspruch 11, wobei die Röhre (27a) auf ihrer in Längsrichtung der Wandung (25) gegenüberliegenden Seite einen den Aerosolaustritt (11) verengenden Rand (28) aufweist.

13. Inhalationsvorrichtung gemäß Anspruch 11 oder Anspruch 12,
wobei der Lufteinlass (13) zumindest teilweise als Unterbrechung in der Röhre (27a) ausgebildet ist.

14. Inhalationsvorrichtung gemäß einem der Ansprüche 10-13,
wobei die in Einschieberichtung durchströmbare Querschnittsfläche des Aerosolaustritts (11) kleiner ist als die frei durchströmbare Fläche neben dem Prallelement (10).

15. Inhalationsvorrichtung gemäß einem der vorangehenden Ansprüche, wobei die Inhalatvorlage (1) einen Druckbehälter (2) aufweist.

## Claims

1. An inhalation device comprising

an inhalate reservoir (1), and
an inhalation actuator (6), comprising:

a receptacle (5) for receiving a discharge nipple (4) of the inhalate reservoir,
an inhalate nozzle (9) for spraying inhalate from at least one nozzle discharge opening of the inhalate nozzle (9),
an inhalate feed (8) for supplying inhalate from the discharge nipple (4) to the inhalate nozzle (9),
an impingement element (10) onto which inhalate can be sprayed from the nozzle discharge opening in a straight line for impact atomization,
an aerosol chamber (12) having an internal volume to which inhalate sprayed from the inhalate nozzle (9) is deliverable,

wherein the aerosol chamber (12) comprises an aerosol outlet (11) for outflow of aerosol from the aerosol chamber (12) and an air supply (13) separate from the inhalate nozzle (9) and the aerosol outlet (11), and the inhalation device is configured such that inhalate exiting the at least one nozzle discharge opening as a liquid jet breaks up freely into droplets prior to impinging on the impingement element (10), and the droplets then meet the impingement element (10) for impact atomization,
**characterized in that** the aerosol outlet (11) comprises a local constriction.

2. The inhalation device according to claim 1,
comprising an insert (21) forming at least one wall of the aerosol chamber (12) and supporting the impingement element (10).

3. The inhalation device according to any of the preceding claims, wherein the receptacle (5), the inhalate feed (8) and the aerosol chamber (12) are made integral with each other.

4. The inhalation device according to any of the preceding claims, wherein the at least one nozzle discharge opening and the aerosol outlet are arranged relative to each other such that inhalate cannot flow in a straight line from the nozzle discharge opening to the aerosol outlet.

5. The inhalation device according to any of the preceding claims, wherein the main discharge direction of the at least one nozzle discharge opening and the main discharge direction of the aerosol outlet (11) are arranged offset relative to one another and/or are at an angle greater than zero and less than 180° relative to one another,
the main discharge direction being defined as a perpendicular to the center of area of the smallest through-flowable area of the nozzle discharge opening or of the aerosol outlet (11), respectively.

6. The inhalation device according to claim 5, wherein the main discharge direction of the at least one nozzle discharge opening and the main discharge direction of the aerosol outlet (11) are at an angle greater than 29° and less than 151° to each other.

7. The inhalation device according to any of the preceding claims, wherein the air supply (13) is provided with an air supply valve (19).

8. The inhalation device according to any of the preceding claims, wherein the maximum extension of the inhalation actuator (2) extends parallel to the discharge nipple (4) inserted into the receptacle (5) as intended.

9. The inhalation device according to any of the preceding claims, wherein at least 10 percent, preferably at least 25 percent, of the internal volume of the aerosol chamber (12) is, with respect to a direction defined by a straight connecting line from the nozzle discharge opening to the center of area of the discharge area of the aerosol outlet (11), located behind the nozzle discharge opening.

10. The inhalation device according to claim 2, wherein the inhalation actuator comprises a mouthpiece (18) disposed in front of the at least one nozzle discharge opening, and
the insert (21) comprises:

centering means for centering the insert (21) in the mouthpiece (18),
a wall (25) arranged transversely to the insertion direction, said wall (25) being arranged in front of the centering means in the insertion direction,
an inhalate inlet opening (24) provided in the wall (25),
the impingement element (10) which is arranged in front of the inhalate inlet opening (24) in the direction opposite to the insertion direction,
an area that is arranged transversely to the insertion direction next to the impingement element (10) and that can be flowed through freely in the insertion direction,
the air inlet (13) arranged, in the insertion direction, in front of the area that can be flowed through freely, and
the aerosol outlet (11) arranged opposite to the direction of insertion in front of the area that can be flowed through freely,
wherein the insert (21) is inserted in the mouthpiece (18) such that a straight flow path is provided from the at least one nozzle discharge opening through the inhalate inlet opening (24) to the impingement element (10).

11. The inhalation device according to claim 10,

wherein the centering means are integrally formed with a continuous or interrupted tube (27a), the longitudinal direction of the tube (27a) corresponding to the insertion direction.

12. The inhalation device according to claim 11, wherein the tube (27a) comprises on its side opposite, in longitudinal direction, of said wall (25) a rim (28) constricting the aerosol outlet (11).

13. The inhalation device according to claim 11 or claim 12,
wherein the air inlet (13) is at least partially formed as an interruption in the tube (27a).

14. The inhalation device according to any of claims 10-13,
wherein the cross-sectional area of the aerosol outlet (11) through which air can flow in the direction of insertion is smaller than the area that can be flowed through freely next to the impingement element (10).

15. The inhalation device according to any of the preceding claims, wherein the inhalate reservoir (1) comprises a pressurized container (2).


**Revendications**

1.   - Dispositif d'inhalation, présentant

- un présentoir de substance à inhaler (1) et
- un actionneur d'inhalation (6), qui présente ce qui suit ;
- un réceptacle (5) pour recevoir un tube de décharge (4) du présentoir de substance à inhaler (1) ;
- une buse de substance à inhaler (9) pour pulvériser la substance à inhaler à partir d'au moins une ouverture de sortie de buse de la buse de substance à inhaler (9) ;
- une amenée (8) de substance à inhaler pour amener la substance à inhaler du tube de décharge (4) à la buse de substance à inhaler (9) ;
- un élément d'impact (10), sur lequel la substance à inhaler peut être pulvérisée en ligne droite à partir de l'ouverture de sortie de buse pour l'atomisation par impact ;
- une chambre d'aérosol (12) avec un volume intérieur, auquel peut être amenée la substance à inhaler pulvérisée par la buse de substance à inhaler (9),
dans lequel la chambre d'aérosol (12) présente une sortie d'aérosol (11) pour le dégagement de l'aérosol à partir de la chambre d'aérosol (12) et une arrivée d'air (13) séparée de la buse de substance à inhaler (9) et de la sortie d'aérosol (11), et
dans lequel le dispositif d'inhalation est conçu de telle sorte que la substance à inhaler sortant de ladite au moins une ouverture de sortie de buse sous forme de jet de liquide se décompose en gouttelettes par décomposition libre avant d'arriver sur l'élément d'impact (10), et que les gouttelettes arrivent ensuite sur l'élément d'impact (10) pour l'atomisation par impact,
**caractérisé par le fait que** la sortie d'aérosol (11) présente un rétrécissement local.

2.   - Dispositif d'inhalation selon la revendication 1, présentant un insert (21), qui forme au moins une paroi de la chambre d'aérosol (12) et porte l'élément d'impact (10).

3.   - Dispositif d'inhalation selon l'une des revendications précédentes, dans lequel le réceptacle (5), l'amenée (8) de substance à inhaler et la chambre d'aérosol (12) sont réalisés conjointement en une seule pièce.

4.   - Dispositif d'inhalation selon l'une des revendications précédentes, dans lequel ladite au moins une ouverture de sortie de buse et la sortie d'aérosol (11) sont disposées l'une par rapport à l'autre de telle sorte que la substance à inhaler ne peut pas s'écouler en ligne droite de la sortie de buse à la sortie d'aérosol (11).

5.   - Dispositif d'inhalation selon l'une des revendications précédentes, dans lequel la direction principale de décharge de ladite au moins une ouverture de sortie de buse et la direction principale de décharge de la sortie d'aérosol (11) sont disposées en étant décalées l'une par rapport à l'autre et/ou forment entre elles un angle supérieur à zéro et inférieur à 180°,
la direction principale de décharge étant définie comme perpendiculaire au centre de gravité superficiel de la plus petite surface pouvant être traversée de l'ouverture de sortie de buse ou de la sortie d'aérosol (11).

**6.** - Dispositif d'inhalation selon la revendication 5, dans lequel la direction principale de décharge de ladite au moins une ouverture de sortie de buse et la direction principale de décharge de la sortie d'aérosol (11) forment entre elles un angle supérieur à 29° et inférieur à 151°.

**7.** - Dispositif d'inhalation selon l'une des revendications précédentes, dans lequel l'admission d'air (13) est équipée d'une soupape d'admission d'air (19).

**8.** - Dispositif d'inhalation selon l'une des revendications précédentes, dans lequel l'étendue maximale de l'actionneur d'inhalation (2) s'étend parallèlement au tube de décharge (4) introduit dans le réceptacle (5) pour l'usage prévu.

**9.** - Dispositif d'inhalation selon l'une des revendications précédentes, dans lequel au moins 10 pour cent, de préférence au moins 25 pour cent, du volume intérieur de la chambre d'aérosol (12) sont disposés derrière l'ouverture de sortie de buse par rapport à une direction qui est définie par une ligne droite reliant l'ouverture de sortie de buse au centre de gravité superficiel de la surface de sortie de la sortie d'aérosol (11).

**10.** - Dispositif d'inhalation selon la revendication 2, dans lequel l'actionneur d'inhalation présente un embout buccal (18) disposé devant ladite au moins une ouverture de sortie de buse, et

dans lequel l'insert (21) présente ce qui suit :

- des moyens de centrage pour centrer l'insert (21) dans l'embout buccal (18) ;
- une paroi (25) disposée transversalement à la direction d'introduction, laquelle est disposée avant les moyens de centrage dans la direction d'introduction, ;
- une ouverture (24) d'entrée de substance à inhaler prévue dans la paroi (25) ;
- l'élément d'impact (10), lequel est disposé avant l'ouverture (24) d'entrée de substance à inhaler à l'opposé de la direction d'introduction ;
- une surface disposée transversalement à la direction d'introduction à côté de l'élément d'impact (10), pouvant être traversée librement dans la direction d'introduction ;
- l'entrée d'air (13) disposée avant la surface pouvant être traversée librement dans la direction d'introduction ; et
- la sortie d'aérosol (11) disposée avant la surface pouvant être traversée librement, dans la direction opposée à la direction d'introduction,

dans lequel l'insert (21) est introduit dans l'embout buccal (18) de telle sorte qu'il existe un trajet d'écoulement en ligne droite de ladite au moins une ouverture de sortie de buse à l'élément d'impact (10) en passant par l'ouverture (24) d'entrée de substance à inhaler.

**11.** - Dispositif d'inhalation selon la revendication 10, dans lequel les moyens de centrage sont réalisés d'un seul tenant avec un tube (27a) continu ou interrompu, la direction longitudinale du tube (27a) correspondant à la direction d'introduction.

**12.** - Dispositif d'inhalation selon la revendication 11, dans lequel le tube (27a) présente, sur son côté opposé à la paroi (25) dans la direction de la longueur, un bord (28) rétrécissant la sortie d'aérosol (11).

**13.** - Dispositif d'inhalation selon la revendication 11 ou la revendication 12, dans lequel l'entrée d'air (13) est formée au moins en partie comme interruption dans le tube (27a).

**14.** - Dispositif d'inhalation selon l'une des revendications 10 à 13, dans lequel la surface en coupe transversale de la sortie d'aérosol (11), pouvant être traversée dans la direction d'introduction, est inférieure à la surface pouvant être traversée librement à côté de l'élément d'impact (10).

**15.** - Dispositif d'inhalation selon l'une des revendications précédentes, dans lequel le présentoir de substance à inhaler (1) présente un réservoir sous pression (2).

*Fig. 1*

*Fig. 2*

Fig. 3

*Fig. 4*

*Fig . 5*

*Fig. 6*

*Fig. 7*

*Fig. 8*

*Fig. 9b*

*Fig. 9c*

*Fig. 9d*

*Fig. 9a*

Fig. 10a

Fig. 10b

Fig. 10c

Fig. 10d

Fig. 10e

Fig. 10f

Flüssigkeitszufuhr mit Druck $P$

Düse

Flüssigkeitsstrahl

Gesamtvolumenstrom

(Primär-) Tropfen

$Z$  $s$

Sprayvolumen-strom

Sprayvolumen-strom

Spray

Prallelement

Ablaufvolumenstrom

Ablaufvolumenstrom

*Fig. 11*

**IN DER BESCHREIBUNG AUFGEFÜHRTE DOKUMENTE**

**In der Beschreibung aufgeführte Patentdokumente**

- US 3069097 A
- US 4940051 A
- WO 2020260903 A1
- US 20130306061 A1
- US 5533498 A
- WO 2020165356 A1
- DE 202021002521 U1